# EUROPÄISCHE PATENTSCHRIFT

(11) **EP 1 419 119 B1**
(45) Veröffentlichungstag und Bekanntmachung des Hinweises auf die Patenterteilung: **03.05.2006**
(21) Anmeldenummer: 02796239.8
(22) Anmeldetag: 17.08.2002
(51) Int. Cl.: C03C 3/076, C03C 3/095, C03C 3/097, C03C 12/00, C03C 4/00

(54) **WASSERUNLÖSLICHES, ANTIMIKROBIELLES SILICATGLAS UND DESSEN VERWENDUNG**
WATER-INSOLUBLE, ANTIMICROBIAL SILICATE GLASS AND USE THEREOF
VERRE DE SILICE ANTIMICROBIEN INSOLUBLE DANS L'EAU ET UTILISATION

(30) Priorität: 22.08.2001 DE 10141117
(43) Veröffentlichungstag der Anmeldung: 19.05.2004
(73) Patentinhaber: Schott AG, 55122 Mainz (DE)
(72) Erfinder: FECHNER, Jörg, Hinrich, 55118 Mainz (DE); ZIMMER, José, 55218 Ingelheim (DE)
(74) Vertreter: Sawodny, Michael-Wolfgang
(86) Internationale Anmeldenummer: PCT/EP2002/009219
(87) Internationale Veröffentlichungsnummer: WO 2003/018495

(56) Entgegenhaltungen:
- EP-A- 0 425 927
- EP-A- 0 921 105
- WO-A-00/76486
- WO-A-96/21628
- JP-A- 7 025 635
- PATENT ABSTRACTS OF JAPAN vol. 1998, no. 14, 31. Dezember 1998 (1998-12-31) & JP 10 231187 A (TOTO LTD), 2. September 1998 (1998-09-02)
- PATENT ABSTRACTS OF JAPAN vol. 1999, no. 13, 30. November 1999 (1999-11-30) & JP 11 228173 A (NIPPON ELECTRIC GLASS CO LTD), 24. August 1999 (1999-08-24)

## Beschreibung

Die Erfindung betrifft ein wasserunlösliches antimikrobielles Silicatglas sowie ein Glaspulver umfassend ein derartiges Silicatglas.

In der vorliegenden Anmeldung wird unter dem Begriff wasserunlöslich verstanden, dass ein Grundglas einer Glaszusammensetzung sich nicht in Wasser auflöst, sondern nur die Oberfläche reaktiv mit dem umgebenden Wasser Ionen austauscht Wasserlösliche antimikrobiell wirkende Silicatgläser sind aus einer Vielzahl von Schriften bekanntgeworden.

So werden in der US 5,290,544 wasserlösliche Gläser für die Anwendungen in kosmetischen Produkten mit sehr geringen SiO₂ und sehr hohen B₂O₃ bzw. hohen P₂O₅-Gehalten beschrieben. Die Gläser weisen Silberkonzentrationen größer 0,5 Gew.-% auf. Diese Gläser besitzen eine extrem niedrige hydrolytische Beständigkeit und neigen dazu, sich in Wasser komplett aufzulösen. Die hierbei freiwerdenden Ag- und/oder Cu-Ionen wirken antibakteriell. Auch in der JP-A-92178433 wird ein wasserlösliches Glaspulver mit SiO₂ < 37 Gew.% als Polymerzusatz mit hohen Silberkonzentrationen > 1 Gew.% beschrieben.

In der US 6,143,318 werden silberhaltige Phosphatgläser beschrieben, die als antimikrobielles Material für die Wundinfektionsbehandlung mit Kombinationen aus Cu, Ag und Zn verwendet werden. Hierbei handelt es sich ebenfalls um wasserlösliche Gläser, die niedrige SiO₂-Konzentrationen und sehr hohe P₂O₅₋Gehalte aufweisen.

Ein weiteres wasserlösliches Glas, das sich vollständig in Wasser auflöst ist beispielsweise ein Glas wie in der JP-A-08245240 beschrieben. Das Glas gemäß der JP-A-08245240 weist einen hohen Phosphorgehalt auf.

Diese Gläser sind aufgrund ihrer niedrigen hydrolytischen Beständigkeit nur sehr beschränkt für eine Mahlung in wäßrigen Medien geeignet.

Silicatgläser ohne antibakterielle Wirkung werden beispielsweise in der DE 2346778 C2 beschrieben. Die DE 2346778 C2 zeigt ein Grundglas ohne AgO und CuO als Emailglas. In der DE 2239307 wird ein Ionenaustauschglas beschrieben, das kein AgO, ZnO, CuO und CeO₂ enthält. Des weiteren zeigt die GB 1294337 eine ionenaustauschbare Glaskeramik, die ebenfalls kein AgO, ZnO, CuO und CeO₂ enthält.

Antimikrobielle silberhaltige Borosilikatgläser bzw. Borophosphatgläser werden in den Schriften JP 10218637, JP 08245240, JP 07291654, JP 03146436, JP 2000264674, JP 2000203876 beschrieben. Diese Gläser weisen größtenteils eine gute hydrolytische Beständigkeit auf und können daher in wäßrigen Medien gemahlen werden..

Zeolithe, die Silber enthalten, das durch Ionenaustausch eingebracht wird, finden ebenfalls als antibakterielles Mittel Verwendung. Dies wird beispielsweise in der US 6,245,732 und WO 0038552 beschrieben.

Schwermetallfreie Gläser, bei denen eine antimikrobielle Wirkung nachgewiesen werden kann, sind in der DE 19932238 und der DE 19932239 beschrieben.

Glaspulver mit einer bioaktiven Wirkung sind aus der WO 97/27148 bekannt. Allerdings dürfen in den aus der WO 97/27148 bekannten Gläsern keine Schwermetalle enthalten sein.

Aus der EP 0 425 927 betrifft eine Keramik und das glasige Vorprodukt hierzu.

Nachteil der im Stand der Technik beschriebenen Gläser ist, daß viele der Substanzen unerwünschte Nebenwirkungen aufweisen und gesundheitlich nicht unbedenklich sind. Sie können allergieauslösend, hautreizend oder in anderer Form belastend für den menschlichen Körper oder die Umwelt sein.

Ein weiterer Nachteil ist, daß die aus dem Stand der Technik bekannten Gläser nur bedingt großtechnisch herstellbar sind.

Desweiteren setzen die aus dem Stand der Technik bekannten Gläser die Ionen sehr schnell frei. Eine kontrollierte Ionenabgabe ist in keiner aus dem Stand der Technik bekannten Schriften beschrieben.

Aufgabe der Erfindung ist es, Gläser bzw. Glaskeramiken bzw. Glaspulver anzugeben, die die Nachteile des Standes der Technik vermeiden. Insbesondere sollen die Gläser im Sinne dieser Anmeldung wasserunlöslich sein und eine antimikrobielle Wirkung aufweisen.

Diese Aufgabe wird durch ein Glas gemäß einem der Anspruche 1 gelöst.

Das erfindungsgemäße Silicatglas kann im großtechnischen Maßstab mit Standardverfahren hergestellt werden.

Die Gläser gemäß der Erfindung können in Pulverform Produkten zugesetzt werden. Daher sind die Gläser vorteilhafterweise für Mahlungen in unterschiedlichen Mahlmedien, zum Beispiel Wasser, geeignet, d. h. das Glas weist eine hinreichende Wasserunlöslichkeit auf.

Das erfindungsgemäße Silicatglaspulver zeigt gegenüber Bakterien, Pilzen sowie Viren eine biozide bzw. eine biostatische Wirkung; ist im Kontakt mit dem Menschen hautverträglich, toxikologisch unbedenklich und insbesondere auch zum Verzehr geeignet.

Aufgrund der Anforderungen an die toxikologische Unbedenklichkeit des Silicatglases sowie deren Eignung zum Verzehr ist das erfindungsgemäße Glas besonders rein. Die Belastung durch Schwermetalle ist gering. So beträgt die Maximalkonzentration im Bereich kosmetischer Produkte vorzugsweise für Pb < 20 ppm, Cd < 5 ppm, As < 5 ppm, Sb < 10 ppm, Hg < 1 ppm, Ni < 10 ppm.

Mit den antimikrobiellen Substanzen kann eine Konservierung der Produkte selbst erzielt oder eine antimikrobielle Wirkung nach außen erreicht werden. Anwendungsbereiche hierfür sind zum Beispiel kosmetische Produkte, Deoprodukte, Lebensmittel, Farben, Lacke, Putze, Papierhygieneprodukte, Medizinprodukte und Reiniger.

Im kosmetischen Bereich spielen Hautirritationen eine wesentliche Rolle. Daher ist es vorteilhaft, wenn der antimikrobielle Glaszusatz besonders hautfreundtich ist.

Ein besonderer Vorteil des erfindungsgemäßen Glaspulvers ist, daß das Glas aufgrund des Schmelz- und Heißformgebungsverhaltens geeignet ist, um in entsprechenden großtechnischen Anlagen hergestellt werden zu können.

Neben der Herstellung über ein Schmelzverfahren sind auch alternative Herstellungsverfahren über die Sol-Gel- oder Reaktionssinter-Route denkbar.

Überraschenderweise ergibt sich bei der erfindungsgemäßen Glaszusammensetzung eine extrem starke antimikrobielle Wirkung des Glases aus einem synergistischen Effekt zwischen der antimikrobiellen Wirkung der Schwermetallionen Ag, Cu, Zn, Ce und der Wirkung durch den Ionenaustausch des Glases. Durch Reaktionen an der Oberfläche des Glases werden Alkalien des Glases durch H+-lonen des wäßrigen Mediums ausgetauscht. Die antimikrobielle Wirkung des lonenaustausches beruht unter anderem auf einer Erhöhung des pH-Wertes und dem osmotischen Effekt auf Mikroorganismen.

Ionenaustauschbare Gläser gemäß der Erfindung wirken in wäßrigen Medien antimikrobiell durch pH-Wert-Erhöhung durch Ionenaustausch zwischen Na bzw. Ca und den H+-lonen der wäßrigen Lösung sowie durch ionenbedingte Beeinträchtigung des Zellwachstums (osmotischer Druck, Störung von Stoffwechselvorgängen der Zellen). Je nach Partikelgröße, Konzentration und der Zusammensetzung des Pulvers werden pH-Werte von bis zu 13 erreicht.

Das Glas enthält SiO₂ als Netzwerkbildner, bevorzugt zwischen 35 bis 70 Gew.-%. Bei niedrigen Konzentrationen nimmt die hydrolytische Beständigkeit stark ab, so daß das Mahlen in wäßrigen Medien nicht mehr ohne signifikante Auflösung des Glases gewährleistet ist Bei höheren Werten kann die Kristallisationsstabilität abnehmen und die Verarbeitungstemperatur wird deutlich erhöht, so daß sich die Schmelz- und Heißformgebungseigenschaften verschlechtern.

Na₂O wird als Flußmittel beim Schmelzen des Glases eingesetzt. Bei Konzentrationen kleiner 5 % wird das Schmelzverhalten negativ beeinflußt Außerdem wirkt der notwendige Mechanismus des Ionenaustausches nicht mehr hinreichend, um eine antibakterielle Wirkung zu erzielen. Bei höheren Na₂O-Konzentrationen als 30 Gew.-% ist eine Verschlechterung der chemischen Resistenz bzw. hydrolytischen Beständigkeit, insbesondere in Verbindung einer Abnahme des SiO₂-Anteils, zu beobachten.

P₂O₅ ist ein Netzwerkbildner und kann die Kristallisationsstabilität erhöhen. Die Konzentrationen sollten nicht oberhalb von 15 Gew.-% liegen, da ansonsten die chemische Beständigkeit des Silicatglases zu stark abnimmt. P₂O₅ verbessert die Oberflächenreaktivität der Gläser.

CaO verbessert die chemische Beständigkeit, insbesondere im leicht alkalischen Bereich und ist daher notwendig, um eine Auflösung des Glases in wäßrigen Medien zu verhindern.

K₂O-Zugaben begünstigen die Austauschbarkeit des Natriums bzw. Kalium kann selber gegen H-+-Ionen ausgetauscht werden.

Die Menge an Al₂O₃ kann zur Erhöhung der chemischen Beständigkeit der Kristallisationsstabilität bis zu maximal 8 Gew.-% hinzugegeben werden. ZnO ist eine wesentliche Komponente für die Heißformgebungseigen-schaften des Glases. Es verbessert die Kristallisationsstabilität und erhöht die Oberflächenspannung. Außerdem kann es den antimikrobiellen Effekt unterstützen. Bei geringen SiO₂-Gehalten erhöht es die Kristatlisations-stabilität. Zur Erzielung einer antimikrobiellen Wirkung können bis zu 8 Gew.-% ZnO enthalten sein. Eine bevorzugte Ausführung enthält < 4.Gew.% ZnO oder < 2 Gew.-%. Ausführungen mit< 1 Gew.-% oder 0,5 Gew.% bzw. < 0,1 Gew.-% sind besonders bevorzugt

AgO, CuO, CeO₂ sind antimikrobiell wirkende Zusätze, die synergistisch die intrinsische antimikrobielle Wirkung des Grundglases verstärken, so daß verhältnismäßig geringe Konzentrationen zugesetzt werden müssen.

Um die gewünschte antimikrobielle Wirkung zu erzielen, enthält das Glas vorzugsweise < 2 Gew.-% oder 1 Gew.-% AgO, CuO, CeO₂. Eine bevorzugte Ausführung enthält < 0,5 Gew.-% oder 0,2 Gew.% AgO, CuO, CeO₂. Eine besonders bevorzugte Ausführung enthält Gehalte von < 0,1 oder 0,01 AgO, CuO, CeO₂ wobei die untere wirksame Menge 0,001 Gew.-% AgO, CuO, CeO₂ beträgt.

Die Summe der Gehalte von AgO, ZnO, CuO und CeO₂liegt < 8 Gew.% oder 5 Gew.-%. In einer bevorzugten Ausführung beträgt die Menge < 2 Gew.-% oder 1 Gew.-%. Eine bevorzugte Ausführung enthält Mengen von < 0,5 oder 2 Gew.-%. Eine besonders bevorzugte Ausführung enthält Gehalte von < 0,1, 0,05 oder 0,01 Gew.-%, wobei als untere wirksame Menge vorzugsweise 0,001 Gew.-% verwendet wird.

Die Summe der Gehalte von AgO, CuO, ZnO und CeO₂ in Gläsern, die mehr als eine dieser Komponenten enthalten, liegt aufgrund synergistischer Effekte jeweils unterhalb den zugesetzten Mengen in Gläsern, die jeweils nur eine dieser Oxidkomponenten enthalten.

Das erfindungsgemäße Glas ruft keine hautirritierenden Wirkungen hervor. Teilweise sind hingegen sogar entzündungshemmende Wirkungen zu beobachten.

Durch eine Kombination der pH-Wirkung und der Ag-Abgabe kann eine erheblichen Steigerung der antimikrobiellen Wirkung erzielt werden, die über die Summe der Einzelwirkungen deutlich hinausgeht Die in das Produkt freigesetzte Konzentration von Ag-Ionen kann hierbei deutlich unter 1 ppm liegen.

Die Einbringung des Ag kann hierbei entweder bereits bei der Schmelze durch entsprechende Silbersalze erfolgen oder aber durch Ionenaustausch des Glases nach der Schmelze.

Zur Erzielung von Farbwirkungen können den Gläsern einzelne oder auch mehrere farbgebende Komponenten wie z.B. Fe₂O₃, CoO, CuO, V₂O₅, Cr₂O₅ in einer Gesamtkonzentration kleiner 4 Gew.-%, vorzugsweise kleiner 1 Gew.-% zugesetzt werden.

Die innerhalb des beanspruchten Zusammi3nsetzungsbereiches liegenden Gläser erfüllen alle Anforderungen bezüglich eines Einsatzes in den Bereichen Papierhygiene, Kosmetik. Farben, Lacken, Putzen, Medizin-produkten, kosmetischen Anwendungen, Nahrungsmittelzusatz sowie Verwendung in Deoprodukten.

Das Glas kann als Glaspulver eingesetzt werden, wobei durch einen Mahlprozeß Partikelgrößen < 100 µm erhalten werden. Als zweckmäßig haben sich Partikelgrößen < 50 µm bzw. < 20 µm erwiesen. Besonders geeignet sind Partikelgrößen < 10 µm sowie kleiner 5 µm. Als ganz besonders geeignet haben sich Partikelgrößen < 1 µm herausgestellt.
Der Mahlprozeß kann sowohl trocken als auch mit wäßrigen und nichtwäßrigen Mahlmedien durchgeführt werden.

Wesentliche Eigenschaft, auch des Glaspulvers, ist die überraschenderweise nachgewiesene Hautverträglichkeit, die auch bei hohen Konzentrationen mit hohen pH-Werten gegeben ist.

Das Glas kann in jeder geeigneten Form einschließlich der genannten Pulverform eingesetzt werden. Mischungen unterschiedlicher Glaspulver aus dem Zusammensetzungsbereich mit unterschiedlichen Zusammensetzungen sind ebenfalls möglich. Die Mischung mit anderen Glaspulvern ist ebenfalls möglich, um bestimmte Effekte zu kombinieren.

Komponenten wie Fluor können je nach Anwendungsgebiet dem Glas bis zu Konzentrationen von in Summe 5 Gew.-% zugesetzt werden.

Die Gesamt-Metallionenabgabe der Gläser zur Erzielung hinreichender biozider bzw. konservierender Wirkungen in einem Produkt kann aufgrund des synergistischen Effektes < 50 ppm, 10 ppm sein, eine bevorzugte Ausführung ergibt < 1 ppm, insbesondere < 1-00 ppb, für pH-Werte zwischen 9 bis 13 pH.

Das in dieser Erfindung beschriebene Glas ist nicht wasserlöslich, sondem wirkt in erster Linie durch Ionenaustausch bzw. lonenabgabe, was mit einer Oberflächenreaktion, pH-Erhöhung und Metallionen-Freisetzung verbunden ist Durch die synergistische antimikrobielle Wirkung des Glases selbst sowie die antimikrobielle Wirkung der Metallionen sind die benötigten Mengen an Metallionen geringer, um eine entsprechende Wirkung zu erzielen.

Die Erfindung soll nachfolgend anhand der Ausführungsbeispiele beschrieben werden.

### Ausführungsbeispiele

Aus den Rohstoffen wurde das Glas in einem Ptatintiegel bei 1600° C erschmozlen und zu Halbzeug oder Ribbons verarbeitet. Die Ribbons wurden in einer Trommelmühle auf Korngrößen von bis zu 4 µm gemahlen. Komgrößen unter 4 µm wurden mit Attritormahlungen in wäßrigem oder nicht wäßrigem Medium erreicht.

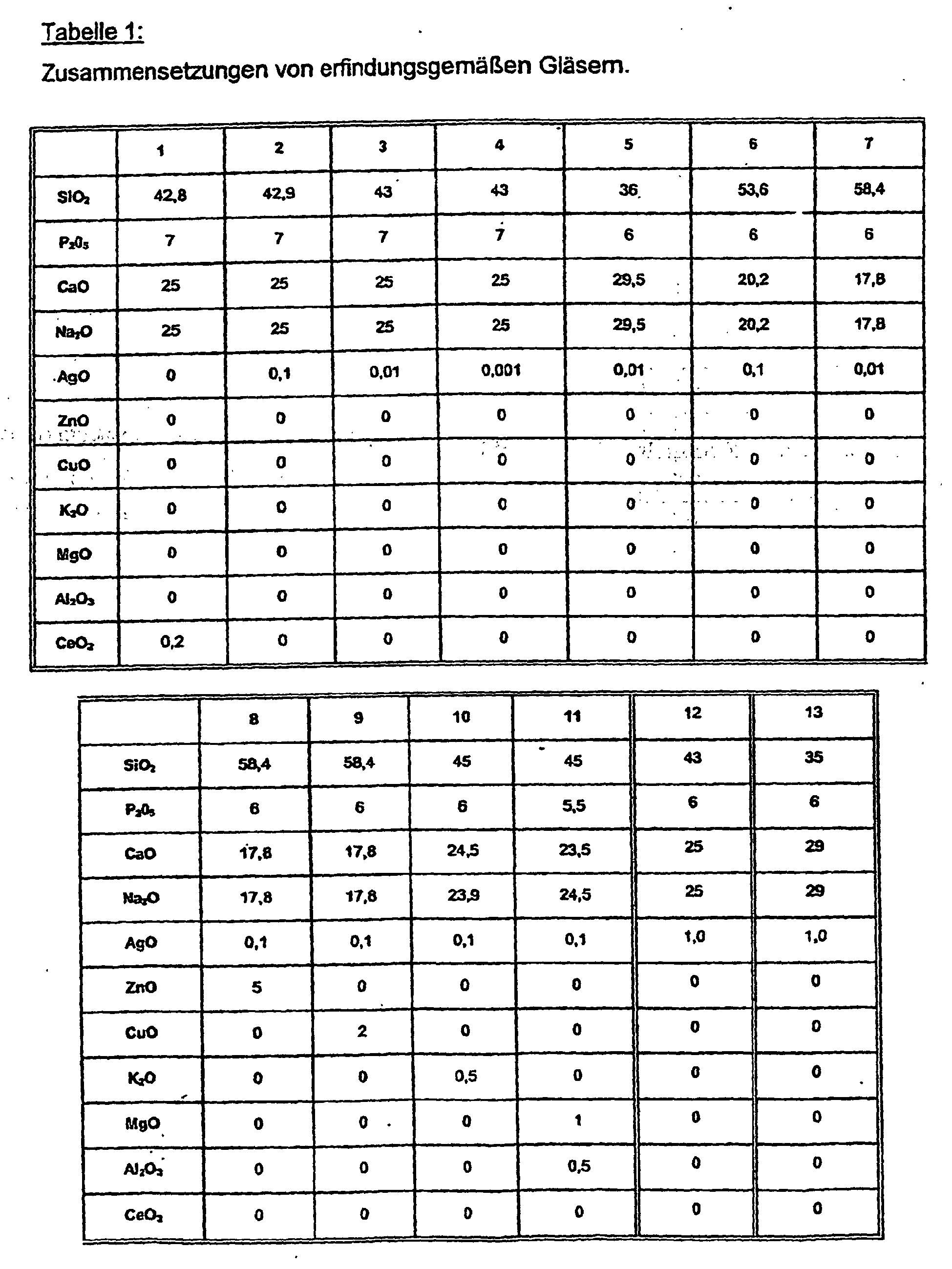

Besonders bevorzugt sind somit Zusammensetzungen erfindungsgemäßer Gläser, die 32 bis 48 Gew-% SiO₂; 5 bis 7 Gew-% P₂O₅; 23 bis 32 Gew-% CaO; 23 bis 32 Gew-% Na₂O; 0,01 bis 2 Gew-% Ag umfassen. Derartige Glaszusammensetzungen haben den Vorteil deutlich-erhöhter Reaktivität. Desweiteren weisen sie eine hohe Wasserunlöslichkeit auf. Durch den in das SiO₂-Netzwerk eingebauten Phosphor wird das Netzwerk so modifiziert, dass sehr leicht Ag freigegeben wird. Ca dient als Stabilisator und vermindert die Löslichkeit des Glases. In den bevorzugten Ausführungsbeispielen ist das Verhältnis von Ca zu Phosphorgehalt so gewählt, dass man eine kontrollierte und langsame Ionenabgabe bei hoher Beständigkeit des Glas erreicht. Besonders bevorzugt beträgt das Verhältnis Ca/P 17/6, bevorzugt 25/6, besonders bevorzugt 30/6. Liegt das Verhältnis des CaIP- Gehaltes somit im Bereich 2,0 - 6,0, bevorzugt im Bereich 2,5 - 5,5, so werden eine kontrollierte und langsame Ionenabgebe bei hoher Beständigkeit des Glases erreicht.

Für Ausführungsbeispiel 2 ergab sich folgende antimikrobielle Wirkung einer 0,1 Gew.% wäßrigen Suspension eines Pulvers aus einem Glas nach Europ. Pharmakopoe (3. Auflage) gemäß Tabelle 2.

**Tabelle 2:**

| | **E.coli** | **P. aeruginosa** | **S. aureus** | **C. albicans** | **A. niger** |
|---|---|---|---|---|---|
| **2 Tage** | 0 | 0 | 0 | 0 | 36000 |
| **7 Tage** | 0 | 0 | 0 | 0 | 100 |
| **14 Tage** | 0 | 0 | 0 | 0 | 0 |
| **21 Tage** | 0 | 0 | 0 | 0 | 0 |
| **28 Tage** | 0 | 0 | 0 | 0 | 0 |

Das Pulver selbst, umfassend ein Glas gemäß Ausführungsbeispiel 2 in nicht suspendierter Form, weist folgende antimikrobielle Wirkung nach Europ. Pharamkopoe (3. Auflage) gemäß Tabelle 3 auf.

**Tabelle 3:**

| | **E.coli** | **P. aeruginosa** | **S. aureus** | **C. albicans** | **A. niger** |
|---|---|---|---|---|---|
| **2 Tage** | 0 | 0 | 0 | 0 | 0 |
| **7 Tage** | 0 | 0 | 0 | 0 | 0 |
| **14 Tage** | 0 | 0 | 0 | 0 | 0 |
| **21 Tage** | 0 | 0 | 0 | 0 | 0 |
| **28 Tage** | 0 | 0 | 0 | 0 | 0 |

Die Silberkonzentration in wäßriger Suspension für ein Pulver, umfassend ein Glas gemäß Ausführungsbeispiel 2, zeigt Tabelle 4 nach verschiedenen Zeiten:

**Tabelle 4:**

| | **0,1% Suspension** | **1% Suspension** | **10% Suspension** |
|---|---|---|---|
| **Ag-Gehalt nach 2 h** | 0,0127 mg/l | < 1 ppm | < 1 ppm |
| **Ag-Gehalt nach 24 h** | < 1 ppm | < 1 ppm | < 1 ppm |

Im Vergleich hierzu zeigt Tabelle 5 die Silberkonzentration in wäßriger Suspension eines Pulvers, umfassend ein Glas gemäß Ausführungsbeispiel 3, nach verschiedenen Zeiten.

**Tabelle 5:**

| | **0,1% Suspension** | **1% Suspension** | **10% Suspension** |
|---|---|---|---|
| **Ag-Gehalt nach 2 h** | < 1 ppm | < 1 ppm | < 1 ppm |
| **Ag-Gehalt nach 24 h** | < 1 ppm | <1 ppm | <1 ppm |

Wie aus den Tabellen 1 bis 5 hervorgeht, besitzen die erfindungsgemäßen Gläser eine antimikrobielle und konservierende sowie stark antimikrobielle Wirkung bei nur geringer Schwermetall-Freisetzung und eine geringe Toxizität.

Damit sind die Gläser hervorragend geeignet als antimikrobieller Zusatz für unterschiedlichste Anwendungen.

## Patentansprüche

1. Wasserunlösliches, antimikrobielles Silicatglasputver, wobei die Glaszusammensetzung die nachfolgenden Komponenten in Gew.-% auf Oxidbasis umfasst
| | |
|---|---|
| SiO₂ | 32 bis 48. |
| Na₂O | 23 bis 32 |
| P₂O₅ | 5 bis 7 |
| CaO | 23 bis.32 |
| AgO | 0,01 bis 2 |
wobei das Verhältnis von Ca/P im Bereich 2,0 bis 6,0 liegt und die Größe der Partikel des Glaspulvers < 100 µm, bevorzugt < 50 µm, besonders bevorzugt < 20 µm sind.

2. Wasserunlösliches, antimikrobielles Silicatglaspulver gemäß Anspruch 1. **dadurch gekennzeichnet, dass** die Zusammensetzung in Gew.-% auf Oxidbasis des weiteren umfasst
| | |
|---|---|
| K₂O | 0 bis 5 |
| ZnO | 0 bis 8 |
| CuO | 0 bis 5 |
| MgO | 0 bis 8 |
| Al₂O₃ | 0 bis 7 |
| CeO₂ | 0 bis 5 |
| Fe₂03 | 0 bis 2, |
wobei die Summe der Komponenten AgO, CuO, CeO₂ > 10 ppm, bevorzugt ≥100 ppm und < 8 Gew.-% ist.

3. Wasserunlösliches, antimikrobielles Silicatglaspulver gemäß Anspruch 1 oder 2, **dadurch gekennzeichnet, dass** das antimikrobielle Silicatglas ZnO im Bereich 5 bis 8 Gew-% auf Oxidbasis enthält

4. Wasserunlösliches, antimikmbielles Silicatglaspulver umfassend ein antimikrobielles Silicatglas gemäß einem der Ansprüche 1 bis 3, **dadurch gekennzeichnet, daß** die Größe der Partikel des Glaspulvers < 5 µm, besonders bevorzugt < 2 µm ist

5. Wasserunlösliches, antimikrobielles Silicatglaspulver nach Anspruch 4, **dadurch gekennzeichnet, daß** Partikel mit einer Größe < 5 µm durch Attritor-Mahlung des Glases in Wasser erhalten werden können.

6. Verwendung eines antimikrobieHen Glaspulvers gemäß einem der Ansprüche 1 bis 5 zur Erzielung einer antimikrobiellen Wirkung.

7. Verwendung eines .antimikrobiellen Glaspulvers gemäß einem der Ansprüche 1 bis 5 zur Erzielung einer antitnikrobiellen und entzündungshemmenden Wirkung

8. Verwendung eines antimikrobiellen Glaspulvers nach einem der Ansprüche 1 bis 5 in der Wundversorgung.

9. Verwendung eines Gtasputvers nach einem der Ansprüche 1 bis 5 in Kosmetikprodukten zur Konservierung.

10. Verwendung eines antimikrobiellen Glaspulvers nach einem der Ansprüche 1 bis 5 in Kosmetikprodukten zur Konservierung und zusätzlich zur Verhinderung von Körpergeruch.

11. Verwendung eines antimikrobiellen Glaspulvers nach einem der Ansprüche 1 bis 5 in Kosmetikprodukten zur Konservierung und zusätzlich verbunden mit einer entzündungshemmenden Wirkung.

12. Verwendung eines antimikrobiellen Glaspulvers nach einem der Ansprüche 1 bis 5 in Deoprodukten zur Konservierung.

13. Verwendung eines antimikrobiellen Glaspulvers nach einem der Ansprüche 1 bis 5 in Oeoprodukten zur Konservierung und zusätzlich zur Verhinderung von Körpergeruch.

14. Verwendung eines antimikrobiellen Glaspulvers nach einem der Ansprüche 1 bis 5 in Deoprodukten zur Konservierung und zusätzlich verbunden mit einer entzündungshemmenden Wirkung.

15. Verwendung eines antimikrobiellen Glaspulvers nach einem der Ansprüche 1 bis 5 in Farben, Lacken und Putzen zur Konservierung.

16. Verwendung eines antimikrobiellen Glaspulvers nach einem der Ansprüche 1 bis 5 in Farben, Lacken und Putzen zur Konservierung, verbunden mit einer Film-Konservierung.

17. Verwendung eines antimikrobiellen Glaspulvers nach einem der Ansprüche 1 bis 5 in Produkten der Papierhygiene zur Vermeidung von Geruchsbildung.

18. Verwendung eines antimikrobiellen Glaspulvers nach einem der Ansprüche 1 bis 5 in Produkten der Papierhygiene zur Vermeidung von Entzündungen der Haut.

19. Verwendung eines antimikrobiellen Glaspulvers nach einem der Ansprüche 1 bis 5 in Nahrungsmitteln zur Konservierung.

20. Verwendung eines antimikrobiellen Glaspulvers nach einem der Ansprüche 1 bis 5 in Nahrungsmitteln zur Konservierung und Desinfektion.

21. Verfahren zur Herstellung eines antimikrobiell wirkenden Glaspulvers nach einem der Anspruche 1 bis 5 umfassend die folgenden Schritte:
21.1 es wird aus Rohstoffen ein Glas erschmolzen;
21.2 das erschmolzene Glas wird zu Ribbons weiterverarbeitet;
21.3 die Ribbons werden zu Glaspulver gemahlen, wobei die Größe der Partikel < 100 µm, bevorzugt < 50 µm, besonders bevorzugt < 20 µm ist.

## Revendications

1. Verre pulvérisé silicieux, insoluble dans l'eau, antimicrobe, dont la composition du verre est constituée des composants suivants en pourcentage en poids sur base oxyde:
| | |
|---|---|
| SiO₂ | 32 à 48 |
| Na₂O | 23 à 32 |
| P₂O₅ | 5 à 7 |
| CaO | 23 à 32 |
| AgO | 0,01 à 2, |
dont la relation de Ca/P est comprise entre 2,0 et 6,0 et dont la taille des particules du verre pulvérisé est < 100 µm, de préférence < 50µm, et particulièrement préferé < 20µm.

2. Verre pulverisé silicieux, insoluble dans l'eau, antimicrobe selon la revendication 1, **caractérisé par** une composition dont le pourcentage en poids sur base oxyde comporte en outre:
| | |
|---|---|
| K₂O | 0 à 5 |
| ZnO | 0 à 8 |
| CuO | 0 à 5 |
| MgO | 0 à 8 |
| Al₂O₃ | 0 à 7 |
| CeO₂ | 0 à 5 |
| Fe₂O₃ | 0 à 2, |
dont la somme des composants AgO, CuO, CeO₂ est > 10 ppm, de préférence > 100 ppm et < 8 pourcentage en poids.

3. Verre pulvérisé silicieux, insoluble dans l'eau, antimicrobe, selon la revendication 1 ou 2, **caractérisé en ce que** le verre silicieux antimicrobe contient ZnO avec un pourcentage en poids sur base oxyde allant de 5 à 8.

4. Verre pulverisé silicieux, insoluble dans l'eau, antimicrobe, comprenant un verre silicieux antimicrobe correspondant aux spécifications 1 à 3, **caractérisé par** une taille des particules de verre pulverisé < 5 µm, de préférence < 2 µm.

5. Verre pulverisé silicieux, insoluble dans l'eau, antimicrobe, selon la revendication 4, **caractérisé par le fait que** les particules d'une taille < 5 µm grâce au broyage à l'attritor du verre peuvent être obtenir dans l'eau.

6. Utilisation d'un verre pulverisé antimicrobe selon l'une des revendications 1 à 5 pour obtenir un effet antimicrobien.

7. Utilisation d'un verre pulverisé antimicrobe selon l'une des revendications 1 à 5 pour obtenir un effet antimicrobien et anti-inflammatoire.

8. Utilisation d'un verre pulverisé antimicrobe selon l'une des revendications 1 à 5 dans les soins des blessures.

9. Utilisation d'un verre pulverisé antimicrobe selon l'une des revendications 1 à 5 dans les produits cosmétiques pour la conservation.

10. Utilisation d'un verre pulverisé antimicrobe selon l'une des revendications 1 à 5 dans les produits cosmétiques pour la conservation et de plus pour éviter les odeurs corporelles.

11. Utilisation d'un verre pulverisé antimicrobe selon l'une des revendications 1 à 5 dans les produits cosmétiques pour la conservation et de plus avec un effet anti-inflammatoire.

12. Utilisation d'un verre pulverisé antimicrobe selon l'une des revendications 1 à 5 dans les produits déodorisants pour la conservation.

13. Utilisation d'un verre pulverisé antimicrobe selon l'une des revendications 1 à 5 dans les produits déodorisants pour la conservation et de plus pour éviter les odeurs corporelles.

14. Utilisation d'un verre pulverisé antimicrobe selon l'une des revendications 1 à 5 dans les produits déodorisants pour la conservation et de plus avec un effet anti-inflammatoire.

15. Utilisation d'un verre pulverisé antimicrobe selon l'une des revendications 1 à 5 dans les peintures, laques et crépis pour la conservation.

16. Utilisation d'un verre pulverisé antimicrobe selon l'une des revendications 1 à 5 dans les peintures, laques et crépis pour la conservation, avec une conservation du feuil.

17. Utilisation d'un verre pulverisé antimicrobe selon l'une des revendications 1 à 5 dans les produits de l'hygiène papier pour éviter les odeurs.

18. Utilisation d'un verre pulverisé antimicrobe selon l'une des revendications 1 à 5 dans les produits de l'hygiène papier pour éviter les inflammations de la peau.

19. Utilisation d'un verre pulverisé antimicrobe selon l'une des revendications 1 à 5 dans les produits alimentaires pour la conservation.

20. Utilisation d'un verre pulverisé antimicrobe selon l'une des revendications 1 à 5 dans les produits alimentaires pour la conservation et la désinfection.

21. Procédé de fabrication d'un verre pulverisé à effet antimicrobe selon l'une des revendications 1 à 5 comprenant les étapes suivantes:
21.1 À partir de matières brutes, un verre est fondu;
21.2 ce verre fondu est traité pour obtenir des ribbons ;
21.3 les ribbons sont moulus pour obtenir du verre pulverisé, à noter que la taille des particules est < 100 µm, de préférence < 50µm, particulièrement préféré < 20 µm.

## Claims

1. Water-insoluble, antimicrobial silicate glass powder, wherein the glass composition comprises the following components in wt.% based on oxides:
| | |
|---|---|
| SiO₂ | 32 to 48 |
| Na₂O | 23 to 32 |
| P₂O₅ | 5 to 7 |
| CaO | 23 to 32 |
| AgO | 0.01 to 2 |
whereby the ratio of Ca/P is in the range from 2.0 to 6.0 and the size of the particles of the glass-powder is < 100 µm, preferably < 50 µm, particularly preferred < 20 µm.

2. Water-insoluble antimicrobial silicate glass powder according to claim 1, **characterized in that** the composition further comprises in wt.% based on oxides:
| | |
|---|---|
| K₂O | 0 to 5 |
| ZnO | 0 to 8 |
| CuO | 0 to 5 |
| MgO | 0 to 8 |
| Al₂O₃ | 0 to 7 |
| CeO₂ | 0 to 5 |
| Fe₂O₃ | 0 to 2 |
whereby the sum of components AgO, CuO, CeO₂ is > 10 ppm, preferably ≥ 100 ppm, and < 8 wt.%.

3. Water-insoluble antimicrobial silicate glass powder according to claim 1 or 2, **characterized in that** the antimicrobial silicate glass contains ZnO in the range from 5 to 8 wt.% based on oxides.

4. Water-insoluble antimicrobial silicate glass powder comprising an antimicrobial silicate glass according to any one of claims 1 to 3, **characterized in that** the size of the particles of the glass powder is < 5 µm, particularly preferred < 2 *µ*m.

5. Water-insoluble antimicrobial silicate glass powder according to claim 4, **characterized in that** particles having a size of < 5 µm may be obtained using attrition grinding of the glass in water.

6. Use of an antimicrobial glass powder according to any one of claims 1 to 5 in order to achieve an antimicrobial effect.

7. Use of an antimicrobial glass powder according to any one of claims 1 to 5 in order to achieve an antimicrobial and an anti-inflammatory effect.

8. Use of an antimicrobial glass powder according to any one of claims 1 to 5 in wound treatment.

9. Use of a glass powder according to any one of claims 1 to 5 for the preservation in cosmetic products.

10. Use of an antimicrobial glass powder according to any one of claims 1 to 5 for the preservation in cosmetic products and additionally for the prevention of body-odour.

11. Use of an antimicrobial glass powder according to any one of claims 1 to 5 for the preservation in cosmetic products and additionally related with an anti-inflammatory effect.

12. Use of an antimicrobial glass powder according to any one of claims 1 to 5 for the preservation in deodorant products.

13. Use of an antimicrobial glass powder according to any one of claims 1 to 5 for the preservation in deodorant products and additionally for the prevention of body-odour.

14. Use of an antimicrobial glass powder according to any one of claims 1 to 5 for the preservation in deodorant products and additionally related with an anti-inflammatory effect.

15. Use of an antimicrobial glass powder according to any one of claims 1 to 5 for the preservation in paints, lacquers and finery.

16. Use of an antimicrobial glass powder according to any one of claims 1 to 5 for the preservation in paints, lacquers and finery related with a film-preservation.

17. Use of an antimicrobial glass powder according to any one of claims 1 to 5 for the prevention of odour-formation in products of paper-hygiene.

18. Use of an antimicrobial glass powder according to any one of claims 1 to 5 for the prevention of skin-inflammation in products of paper-hygiene.

19. Use of an antimicrobial glass powder according to any one of claims 1 to 5 for the preservation in foods.

20. Use of an antimicrobial glass powder according to any one of claims 1 to 5 for the preservation and disinfection in foods.

21. Process for the manufacturing of an antimicrobial effective glass powder according to any one of claims 1 to 5, comprising the following steps:
21.1 a glass is molten from raw materials;
21.2 the molten glass is processed to ribbons;
21.3 the ribbons are molten into glass powder, wherein the size of the particles is < 100 µm, preferably < 50 µm, particularly preferred < 20 µm.
